(19) Europäisches Patentamt
European Patent Office
Office européen des brevets

(11) EP 0 815 093 B1

(12) **EUROPÄISCHE PATENTSCHRIFT**

(45) Veröffentlichungstag und Bekanntmachung des Hinweises auf die Patenterteilung:
**16.08.2000 Patentblatt 2000/33**

(21) Anmeldenummer: **96905830.4**

(22) Anmeldetag: **01.03.1996**

(51) Int Cl.[7]: **C07D 285/04**, C07D 285/08, C07D 417/12, A01N 43/82, A01N 43/836

(86) Internationale Anmeldenummer:
**PCT/EP96/00836**

(87) Internationale Veröffentlichungsnummer:
**WO 96/28434 (19.09.1996 Gazette 1996/42)**

(54) **HERBIZIDE FLUORTHIADIAZOLYLOXYACETAMIDE**

HERBICIDE FLUOROTHIADIAZOLYL OXYACETAMIDES

FLUORTHIADIAZOLYLOXYACETAMIDES HERBICIDES

(84) Benannte Vertragsstaaten:
**DE FR IT**

(30) Priorität: **14.03.1995 DE 19509044**

(43) Veröffentlichungstag der Anmeldung:
**07.01.1998 Patentblatt 1998/02**

(73) Patentinhaber: **BAYER AG**
**51368 Leverkusen (DE)**

(72) Erfinder:
- **FÖRSTER, Heinz**
  **D-56337 Kadenbach (DE)**
- **BERTSCH, Achim**
  **D-51061 Köln (DE)**
- **BÖHM, Stefan**
  **D-47800 Krefeld (DE)**
- **DIEHR, Hans-Joachim**
  **D-42329 Wuppertal (DE)**
- **KYSELA, Ernst**
  **D-51427 Bergisch Gladbach (DE)**
- **DOLLINGER, Markus**
  **D-51381 Leverkusen (DE)**
- **SANTEL, Hans-Joachim**
  **D-51371 Leverkusen (DE)**

(56) Entgegenhaltungen:
**EP-A- 0 192 117** **EP-A- 0 300 344**

Bemerkungen:
Die Akte enthält technische Angaben, die nach dem Eingang der Anmeldung eingereicht wurden und die nicht in dieser Patentschrift enthalten sind.

EP 0 815 093 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

**Beschreibung**

Die Erfindung betrifft neue Fluorthiadiazolyloxyacetamide, ein Verfahren und ein neues Zwischenprodukt zu ihrer Herstellung und ihre Verwendung als Herbizide.

Es ist bereits bekannt, daß bestimmte Chlorthiadiazolyloxyacetamide, wie z.B. die Verbindung N-Methyl-N-phenyl-2-(3-Chlor-1,2,4-thiadiazol-5-yl-oxy)-acetamid, herbizide Eigenschaften aufweisen (vgl. EP-A 300344). Die Wirksamkeit dieser vorbekannten Verbindungen ist jedoch, insbesondere bei niedrigen Aufwandmengen und Konzentrationen, nicht in allen Anwendungsgebieten völlig zufriedenstellend.

Es wurden erfindungsgemäß die folgenden, neuen Fluorthiadiazolyloxyacetamide gefunden:

N-Methyl-N-phenyl-2-(3-fluor-1,2,4-thiadiazol-5-yl-oxy)-acetamid der Formel (Ia-1)

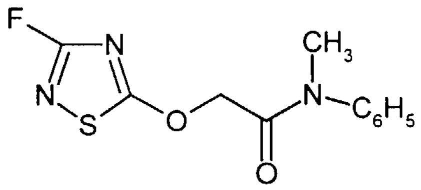

(Ia-1)

sowie die (3-Fluor-1,2,4-thiadiazol-5-yl-oxy)-acetamide der Formel (Ia)

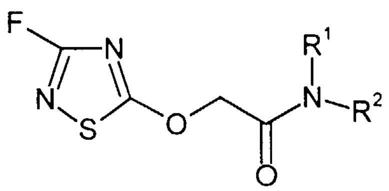

(Ia)

worin $R^1$ und $R^2$ jeweils für die in der folgenden Tabelle genannten Reste stehen:

**Tabelle 1**

| Nr. | $R^1$ | $R^2$ |
|---|---|---|
| Ia-2 | $-(CH_2)_6-$ | |
| Ia-3 | $CH_2$-$CH=CH_2$ | $CH_2$-$CH=CH_2$ |
| Ia-4 | $CH(CH_3)_2$ | $OCH_2CH_2OC_2H_5$ |
| Ia-5 | $CH(CH_3)_2$ | 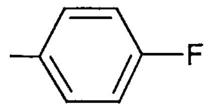 |
| Ia-6 | 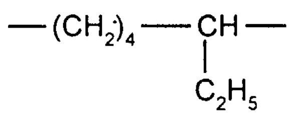 | |
| Ia-7 | $CH_3$ | 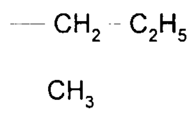 |
| Ia-8 | n-$C_3H_7$ | n-$C_3H_7$ |
| Ia-9 | $-(CH_2)_5-$ | |
| Ia-10 | $CH_2$-$CH=CH_2$ | $CH_2$-$CH=CH_2$ |
| Ia-11 | $CH(CH_3)_2$ | $OCH(CH_3)_2$ |
| II-12 | $CH_3$ | 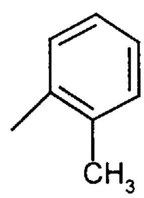 |
| Ia-13 | $C_2H_5$ | $C_2H_5$ |
| Ia-14 | $C_2H_5$ | 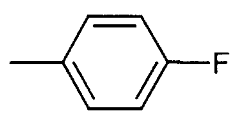 |
| Ia-15 | | 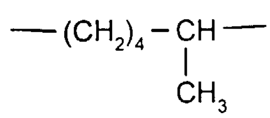 |
| Ia-16 | | 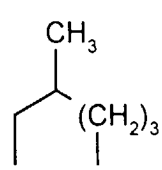 |

**Tabelle 1** (Fortsetzung)

| Nr. | $R^1$ | $R^2$ |
|---|---|---|
| Ia-17 | $CH_3$ | n-$C_4H_9$ |
| Ia-18 | $CH_3$ | 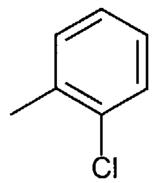 |
| Ia-19 | $C_2H_5$ | $C_6H_5$ |
| Ia-20 | n-$C_4H_9$ | n-$C_4H_9$ |
| Ia-21 | $CH_3$ | 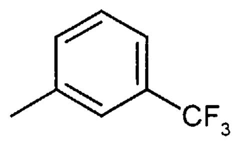 |
| Ia-22 | $CH_3$ | 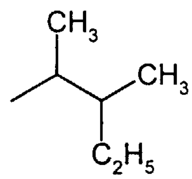 |
| Ia-23 | 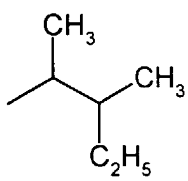 | $OCH_3$ |
| Ia-24 | $CH_3$ | 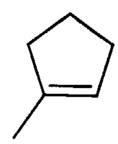 |
| Ia-25 | $CH_3$ | 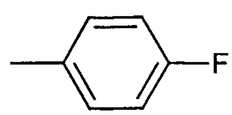 |
| Ia-26 | $CH_2CH_2OC_2H_5$ | $CH_2CH_2OC_2H_5$ |
| Ia-27 | 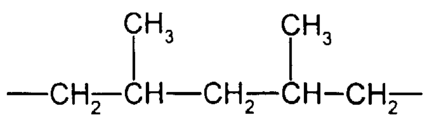 | |

**Tabelle 1** (Fortsetzung)

| Nr. | $R^1$ | $R^2$ |
|---|---|---|
| Ia-28 | $CH_3$ | $CH_2C_6H_5$ |
| Ia-29 | $CH_3$ | 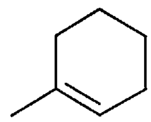 |
| Ia-30 | $CH_3$ | 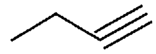 |
| Ia-31 | $CH(CH_3)_2$ | 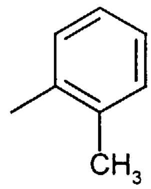 |
| Ia-32 | $CH_3$ | 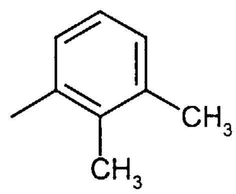 |
| Ia-33 | | 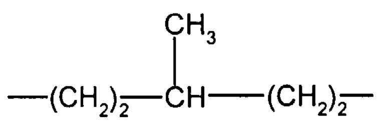 |
| Ia-34 | $C_2H_5$ | 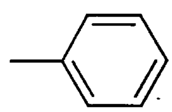 |
| Ia-35 | | 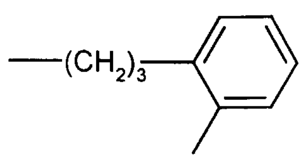 |
| Ia-36 | | 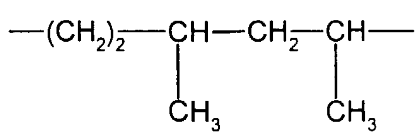 |
| Ia-37 | | 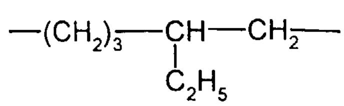 |

**Tabelle 1** (Fortsetzung)

| Nr. | $R^1$ | $R^2$ |
|---|---|---|
| Ia-38 | $CH(CH_3)_2$ | 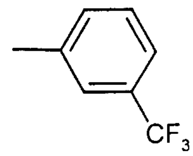 |
| Ia-39 | $CH(CH_3)_2$ | 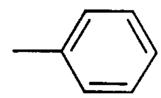 |
| Ia-40 | $CH(CH_3)_2$ | 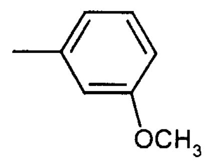 |

Weiter wurde gefunden, daß man die neuen Fluorthiadiazolyloxyacetamide der allgemeinen Formel (I) erhält, wenn man 3-Fluor-5-methylsulfonyl-1,2,4-thiadiazol der Formel (II-1)

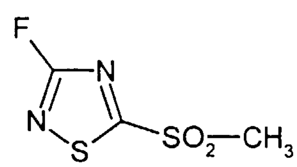

(II-1)

mit Hydroxyacetamiden der allgemeinen Formel (III)

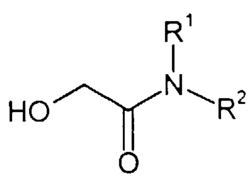

(III),

in welcher

$R^1$ und $R^2$ die oben angegebene Bedeutung haben,

gegebenenfalls in Gegenwart eines Verdünnungsmittels, gegebenenfalls in Gegenwart eines Säurebindemittels und gegebenenfalls in Gegenwart eines Katalysators umsetzt.

Schließlich wurde gefunden, daß die neuen Fluorthiadiazolyloxyacetamide der Formeln (Ia) und (Ia-1) interessante herbizide Eigenschaften besitzen.

Überraschenderweise zeigen die erfindungsgemäßen Fluorthiadiazolyloxyacetamide der allgemeinen Formeln (Ia) und (Ia-1) bei teilweise guter Kulturpflanzen-Verträglichkeit erheblich stärkere herbizide Wirksamkeit als die

aus dem Stand der Technik bekannte Verbindung N-Methyl-N-phenyl-2-(3-chlor-1,2,4-thiadiazol-5-yl-oxy)-acetamid.

Die beim erfindungsgemäßen Verfahren zur Herstellung der Verbindungen der Formeln (Ia) oder (Ia-1) als Ausgangsstoff zu verwendende Alkylsulfonylverbindung ist durch die Formel (II-1) definiert.

Die Ausgangsverbindung der Formel (II-1) ist aus der EP-A 300 344 bekannt.

Man erhält die Alkylsulfonylverbindung der Formel (II-1), wenn man eine entsprechende Alkylthioverbindung der Formel (IV)

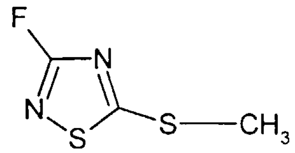

(IV)

mit einem Oxidationsmittel, wie z.B. Hydrogenperoxid, gegebenenfalls in Gegenwart eines Katalysators, wie z.B. Natriumwolframat, und gegebenenfalls in Gegenwart eines Verdünnungsmittels, wie z.B. Essigsäure und/oder Wasser, bei Temperaturen zwischen 0°C und 100°C umsetzt (vgl die Herstellungsbeispiele).

Die als Vorprodukte benötigte Methylthioverbindung der Formel (IV) ist noch nicht aus der Literatur bekannt; sie ist als neuer Stoff ebenfalls Gegenstand der vorliegenden Anmeldung.

Man erhält die neue Verbindung der Formel (IV), wenn man das entsprechende Amino-methylthio-thiadiazol mit Natriumnitrit und Hydrogenfluorid in Gegenwart von Pyridin bei Temperaturen zwischen -30°C und +50°C umsetzt, oder wenn man das entsprechende Chlor-methylthio-thiadiazol mit Kaliumfluorid in Gegenwart eines Katalysators, wie z.B. 18-Krone-6, in Gegenwart eines Verdünnungsmittels, wie z.B. Sulfolan, bei Temperaturen zwischen 100°C und 180°C umsetzt (vgl. die Herstellungsbeispiele).

Die beim erfindungsgemäßen Verfahren zur Herstellung der Verbindungen der Formeln (Ia) oder (Ia-1) weiter als Ausgangsstoffe zu verwendenden Hydroxyacetamide sind durch die Formel (III) allgemein definiert. Dabei haben $R^1$ und $R^2$ die in der oben aufgeführten Tabelle beschriebene Bedeutung.

Die Hydroxyacetamide der Formel (III) sind bekannt und/oder können nach an sich bekannten Verfahren hergestellt werden (vgl. US 4509971, US 4645525, US 4334073, DE 3038598, DE 3038636, EP 37526, EP 348737, DE 3819477).

Als Verdünnungsmittel zur Durchführung des erfindungsgemäßen Verfahrens kommen die üblichen inerten organischen Lösungsmittel in Betracht. Hierzu gehören insbesondere aliphatische, alicyclische oder aromatische, gegebenenfalls halogenierte Kohlenwasserstoffe, wie beispielsweise Pentan, Hexan, Heptan, Petrolether, Ligroin, Benzin, Benzol, Toluol, Xylol, Chlorbenzol, Dichlorbenzol, Cyclohexan, Methylcyclohexan, Dichlormethan, Chloroform, Tetrachlormethan; Ether, wie Diethylether, Diisopropylether, t-Butyl-methylether, t-Pentyl-methylether, Dioxan, Tetrahydrofuran, Ethylenglykol-dimethyl- oder -diethylether, Diethylenglykol-dimethylether oder -diethylether; Ketone, wie Aceton, Butanon oder Methyl-isobutyl-keton; Nitrile, wie Acetonitril, Propionitril, Butyronitril oder Benzonitril; Amide, wie N,N-Dimethylformamid, N,N-Dimethylacetamid, N-Methylformanilid, N-Methyl-pyrrolidon oder Hexamethylphosphorsäuretriamid; Ester wie Essigsäuremethylester, -ethylester, -n- oder -i-propylester, -n-, -i- oder -s-butylester, Sulfoxide, wie Dimethylsulfoxid; Alkohole, wie Methanol, Ethanol, n- oder i-Propanol, n-, i-, s- oder t-Butanol, Ethylenglykol-monomethylether oder -monoethylether, Diethylenglykolmonomethylether, Diethylenglykolmonoethylether; deren Gemische mit Wasser oder reines Wasser.

Das erfindungsgemäße Verfahren wird vorzugsweise in Gegenwart eines geeigneten Säurebindemittels durchgeführt. Als solche kommen alle üblichen anorganischen oder organischen Basen infrage. Hierzu gehören beispielsweise Alkalimetall- oder Erdalkalimetallhydride, -hydroxide, -amide, -alkoholate, -acetate, -carbonate oder -hydrogencarbonate, wie beispielsweise Lithium-, Natrium-, Kalium- oder Calcium-hydrid, Lithium-, Natrium- oder Kalium-amid, Natrium- oder Kalium-methylat, Natrium- oder Kalium-ethylat, Natrium- oder Kalium-propylat, Aluminiumisopropylat, Natrium- oder Kalium-tert-butylat, Natrium- oder Kalium-hydroxid, Ammoniumhydroxid, Natrium-, Kalium- oder Calcium-acetat, Ammoniumacetat, Natrium-, Kalium- oder Calciumcarbonat, Ammoniumcarbonat, Natrium- oder Kalium-hydrogencarbonat, sowie basische organische Stickstoffverbindungen, wie Trimethylamin, Triethylamin, Tripropylamin, Tributylamin, Ethyl-diisopropylamin, N,N-Dimethylcyclohexylamin, Dicyclohexylamin, Ethyl-dicyclohexylamin, N.N-Dimethylanilin, N,N-Dimethyl-benzylamin, Pyridin, 2-Methyl-, 3-Methyl- und 4-Methyl-pyridin, 2,4-Dimethyl-, 2,6-Dimethyl-, 3,4-Dimethyl- und 3,5-Dimethyl-pyridin, 5-Ethyl-2-methyl-pyridin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens in einem größeren Bereich variiert werden. Im allgemeinen arbeitet man bei Temperaturen zwischen -50°C und +100°C, vorzugsweise bei Temperaturen zwischen -20°C und +60°C.

Das erfindungsgemäße Verfahren wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis. Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen und dikotylen Kulturen vor allem im Vorauflauf-Verfahren.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur-und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat,

sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide in Frage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4-D, 2,4-DB, 2,4-DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofopethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuronethyl, Chlorsulfuron, Cinosulfuron, Metsulfuron-methyl, Nicosulfuron, Primisulfuron, Pyrazosulfuron-ethyl, Thifensulfuronmethyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

**Herstellungsbeispiele:**

**Beispiel 1**

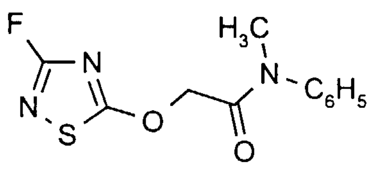

3,6 g (19 mMol) 3-Fluor-5-methylsulfonyl-1,2,4-thiadiazol und 3,1 g (19 mMol) N-Methyl-N-phenyl-hydroxyacetamid werden in 30 ml Aceton vorgelegt und die Mischung wird auf -20°C abgekühlt. Dann wird eine Lösung von

0,76 g Natriumhydroxid in 3 ml Wasser tropfenweise dazu gegeben. Die Reaktionsmischung wird 12 Stunden bei -15°C gerührt, dann mit Wasser auf etwa das doppelte Volumen verdünnt und das kristallin angefallene Produkt durch Absaugen isoliert.

Man erhält 2,6 g (52% der Theorie) N-Methyl-N-phenyl-2-(3-fluor-1,2,4-thiadiazol-5-yl-oxy)-acetamid vom Schmelzpunkt 82°C.

Analog Beispiel 1 sowie entsprechend der allgemeinen Beschreibung des erfindungsgemäßen Herstellungsverfahrens können beispielsweise auch die in der nachstehenden Tabelle 2 aufgeführten Verbindungen der Formel (Ia) - wobei $R^3$ in jedem Einzelfall für 3-Fluor-1,2,4-thiadiazol-5-yl steht - hergestellt werden.

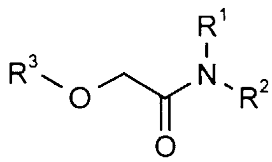

(Ia)

**Tabelle 2:** Beispiele für die Verbindungen der Formel (Ia)

| Bsp.-Nr. | $R^1$ | $R^2$ | Physikal. Daten |
|---|---|---|---|
| 2 | $-(CH_2)_6$ | | $n_{20}^{D}$ = 1,5102 |
| 3 | $CH_2$-$CH=CH_2$ | $CH_2$-$CH=CH_2$ | $n_{20}^{D}$ = 1,5058 |
| 4 | $CH(CH_3)_2$ | $OCH_2CH_2OC_2H_5$ | $n_{20}^{D}$ = 1,4748 |
| 5 | $CH(CH_3)_2$ | 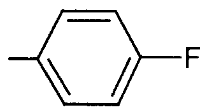 | Fp.: 50°C |
| 6 | 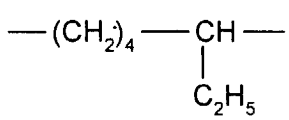 | | $n_{20}^{D}$ = 1,5201 |
| 7 | $CH_3$ | 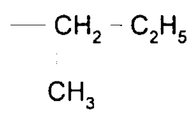 | $n_{20}^{D}$ = 1,4949 |
| 8 | n-$C_3H_7$ | n-$C_3H_7$ | $n_{20}^{D}$ = 1,4945 |
| 9 | $-(CH_2)_5-$ | | |
| 10 | $CH_2$-$CH=CH_2$ | $CH_2$-$CH=CH_2$ | |
| 11 | $CH(CH_3)_2$ | $OCH(CH_3)_2$ | $n_{20}^{D}$ = 1,4808 |
| 12 | $CH_3$ | 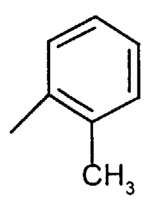 | $n_{20}^{D}$ = 1,5423 |
| 13 | $C_2H_5$ | $C_2H_5$ | $n_{20}^{D}$ = 1,4983 |
| 14 | $C_2H_5$ | 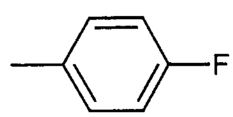 | |
| 15 | 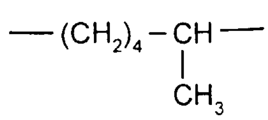 | | $n_{20}^{D}$ = 1,592 |

**Tabelle 2:** (Fortsetzung)

| Bsp.-Nr. | $R^1$ | $R^2$ | Physikal. Daten |
|---|---|---|---|
| 16 | | 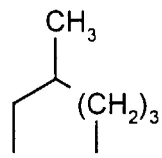 | $n_{20}^{D}$ = 1,5173 |
| 17 | $CH_3$ | n-$C_4H_9$ | $n_{20}^{D}$ = 1,4960 |
| 18 | $CH_3$ | 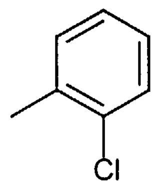 | |
| 19 | $C_2H_5$ | $C_6H_5$ | |
| 20 | n-$C_4H_9$ | n-$C_4H_9$ | $n_{20}^{D}$ = 1,4941 |
| 21 | $CH_3$ | 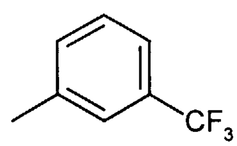 | Fp.: 48°C |
| 22 | $CH_3$ | 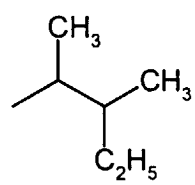 | $n_{20}^{D}$ = 1,4867 |
| 23 | 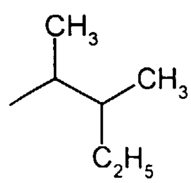 | $OCH_3$ | |
| 24 | $CH_3$ | 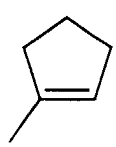 | |
| 25 | $CH_3$ | 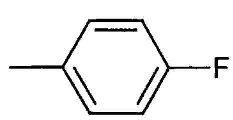 | |

**Tabelle 2:** (Fortsetzung)

| Bsp.-Nr. | $R^1$ | $R^2$ | Physikal. Daten |
|---|---|---|---|
| 26 | $CH_2CH_2OC_2H_5$ | $CH_2CH_2OC_2H_5$ | |
| 27 | 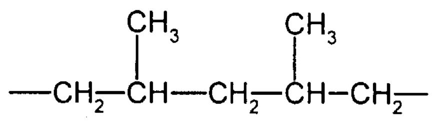 | | |
| 28 | $CH_3$ | $CH_2C_6H_5$ | |
| 29 | $CH_3$ | 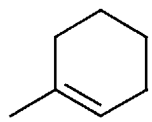 | $n_{20}^{D} = 1,5278$ |
| 30 | $CH_3$ | 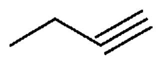 | |
| 31 | $CH(CH_3)_2$ | 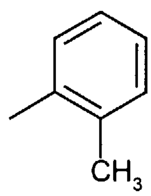 | |
| 32 | $CH_3$ | 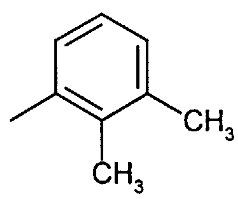 | |
| 33 | 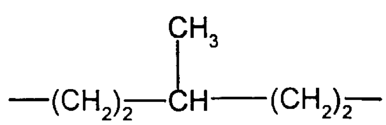 | | $n_{20}^{D} = 1,5191$ |
| 34 | $C_2H_5$ | 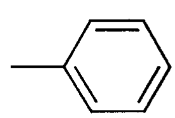 | Fp.: 40°C |
| 35 | 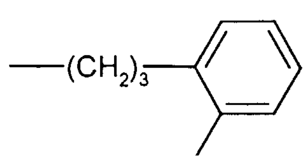 | | $n_{20}^{D} = 1,5870$ |

**Tabelle 2:** (Fortsetzung)

| Bsp.-Nr. | $R^1$ | $R^2$ | Physikal. Daten |
|---|---|---|---|
| 36 | | 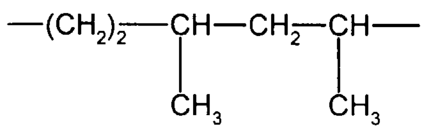 | $n_{20}^{D}$ = 1,5105 |
| 37 | | 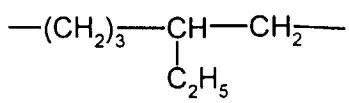 | $n_{20}^{D}$ = 1,5136 |
| 38 | $CH(CH_3)_2$ | 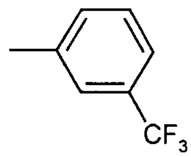 | Fp.: 40°C |
| 39 | $CH(CH_3)_2$ | 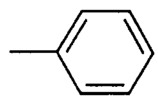 | $n_{20}^{D}$ = 1,5212 |
| 40 | $CH(CH_3)_2$ | 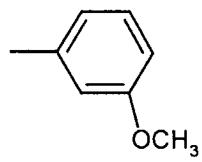 | Fp.: 85°C |

**Ausgangsstoffe der Formel (II):**

**Beispiel (II-1)**

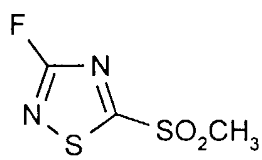

Eine Mischung aus 22,3 g (0,15 Mol) 3-Fluor-5-methylthio-1,2,4-thiadiazol, 0,3 g Natriumwolframat und 120 ml Essigsäure wird auf 70°C erhitzt und bei dieser Temperatur werden 48 ml (0,44 Mol $H_2O_2$) einer wässrigen Lösung von Hydrogenperoxid tropfenweise dazu gegeben. Dann wird die Mischung mit 400 ml Wasser verdünnt und mit Chloroform geschüttelt. Die organische Phase wird dann abgetrennt, mit 5%iger wässriger Natriumhydrogensulfit-Lösung gewaschen, mit Natriumsulfat getrocknet und filtriert. Vom Filtrat wird dann das Lösungsmittel im Wasserstrahlvakuum sorgfältig abdestilliert.

Man erhält 20 g (77% der Theorie) 3-Fluor-5-methylsulfonyl-1,2,4-thiadiazol als blaßgelben, öligen Rückstand vom Brechungsindex $n_{20}^{D}$ = 1,5430.

**Ausgangsstoffe der Formel (IV):**

**Beispiel (IV-1)**

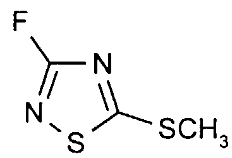

1240 ml (61,2 Mol) Hydrogenfluorid werden bei 0°C bis 10°C vorgelegt und unter Kühlen mit 660 ml (8,14 Mol) Pyridin versetzt. Dann werden bei 10°C 100 g (0,68 Mol) 3-Amino-5-methylthio-1,2,4-thiadiazol dazu gegeben und die Mischung wird anschließend auf -15°C abgekühlt. Bei -15°C bis -5°C werden dann 49,2 g (0,72 Mol) Natriumnitrit innerhalb von ca. 3 Stunden portionsweise dazu gegeben. Die Temperatur der Mischung wird dann langsam von ca. -10°C auf +40°C angehoben, wobei Stickstoff freigesetzt wird. Nach Abklingen der Stickstoffentwicklung wird die Mischung auf 2,5 Liter Wasser gegossen und dann viermal mit je 250 ml Cyclohexan extrahiert. Die vereinigten Extraktionslösungen werden mit wässriger Natriumhydrogencarbonat-Lösung gewaschen, mit Natriumsulfat getrocknet und filtriert. Das Filtrat wird im Wasserstrahlvakuum eingeengt und der Rückstand im Hochvakuum über eine Vigreux-Kolonne (10 cm) fraktioniert.

Man erhält 34 g (33% der Theorie) 3-Fluor-5-methylthio-1,2,4-thiadiazol vom Siedepunkt 62°C/0,3mbar.

$^1$H-NMR ($CDCl_3$, δ): 2,73 ppm.

**Anwendungsbeispiele:**

In den Anwendungsbeispielen wird die nachstehend genannte Verbindung als Vergleichssubstanz verwendet.

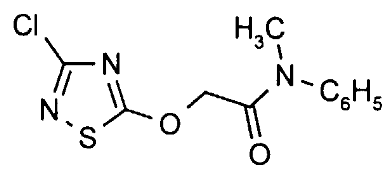

N-Methyl-N-phenyl-2-(3-chlor-1,2,4-thiadiazol-5-yl-oxy)-acetamid (bekannt aus EP-A 300344).

**Beispiel A**

| Pre-emergence-Test | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach 24 Stunden wird der Boden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0 % = | keine Wirkung (wie unbehandelte Kontrolle) |
| 100 % = | totale Vernichtung |

In diesem Test zeigen beispielsweise die Verbindungen gemäß den Herstellungsbeispielen 1, 2, 3 und 4 bei teilweise guter Verträglichkeit gegenüber Kulturpflanzen, wie z.B. Gerste und Soja, erheblich stärkere Wirkung gegen Unkräuter als die bekannte Verbindung A.

**Tabelle A: Pre-emergence-Test/Gewächshaus**

| Wirkstoff | Aufwand-menge (g/ha) | Ger-ste | Soja | Agro-pyron | Ave-na | Bro-mus | Ama-ran-thus | Matri-caria | Sola-num |
|---|---|---|---|---|---|---|---|---|---|
| 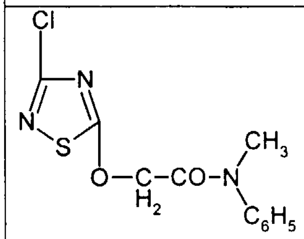 (A) (bekannt) | 500 | 40 | 40 | 60 | 60 | 40 | 70 | 70 | 20 |
| 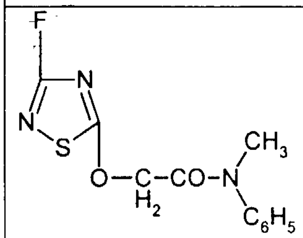 (1) | 500 | 20 | 20 | 95 | 95 | 95 | 100 | 100 | 95 |
| 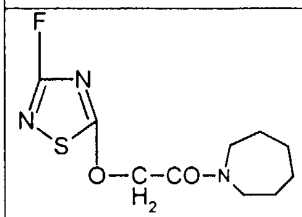 (2) | 500 | 50 | 0 | 95 | 90 | 70 | 100 | 95 | 95 |
| 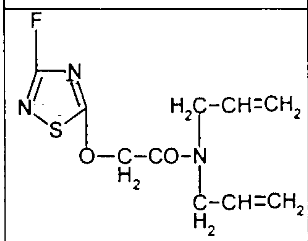 (3) | 500 | - | 0 | 100 | 100 | 95 | 100 | 90 | 80 |
| 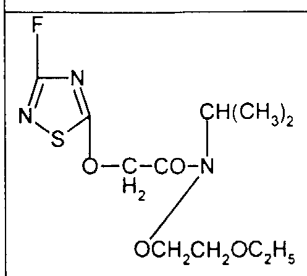 (4) | 500 | - | 60 | 100 | 100 | 100 | 100 | 90 | 70 |

## Patentansprüche

1. N-Methyl-N-phenyl-2-(3-fluor-1,2,4-thiadiazol-5-yl-oxy)-acetamid der Formel (Ia-1)

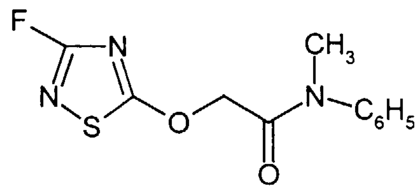

(Ia-1)

sowie die (3-Fluor-1,2,4-thiadiazol-5-yl-oxy)-acetamide der Formel (Ia)

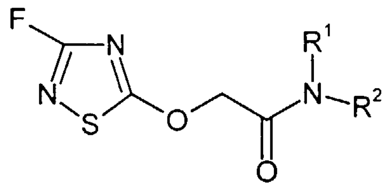

(Ia)

worin $R^1$ und $R^2$ jeweils für die in der folgenden Tabelle genannten Reste stehen:

**Tabelle**

| Nr. | $R^1$ | $R^2$ |
|---|---|---|
| Ia-2 | $-(CH_2)_6-$ | |
| Ia-3 | $CH_2-CH=CH_2$ | $CH_2-CH=CH_2$ |
| Ia-4 | $CH(CH_3)_2$ | $OCH_2CH_2OC_2H_5$ |
| Ia-5 | $CH(CH_3)_2$ | 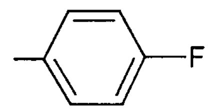 |
| Ia-6 | 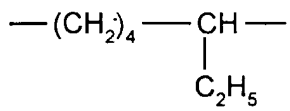 | |
| Ia-7 | $CH_3$ | 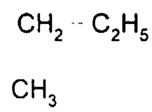 |
| Ia-8 | $n-C_3H_7$ | $n-C_3H_7$ |
| Ia-9 | $-(CH_2)_5-$ | |
| Ia-10 | $CH_2-CH=CH_2$ | $CH_2-CH=CH_2$ |
| Ia-11 | $CH(CH_3)_2$ | $OCH(CH_3)_2$ |
| II-12 | $CH_3$ | 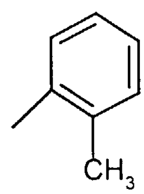 |
| Ia-13 | $C_2H_5$ | $C_2H_5$ |
| Ia-14 | $C_2H_5$ | 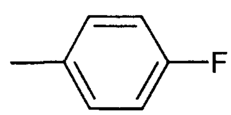 |
| Ia-15 | | 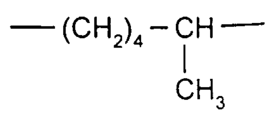 |
| Ia-16 | | 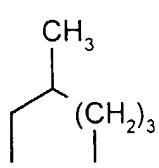 |

**Tabelle** (Fortsetzung)

| Nr. | $R^1$ | $R^2$ |
|---|---|---|
| Ia-17 | $CH_3$ | n-$C_4H_9$ |
| Ia-18 | $CH_3$ | 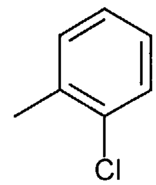 |
| Ia-19 | $C_2H_5$ | $C_6H_5$ |
| Ia-20 | n-$C_4H_9$ | n-$C_4H_9$ |
| Ia-21 | $CH_3$ | 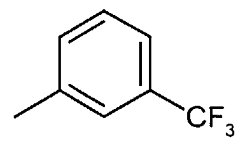 |
| Ia-22 | $CH_3$ | 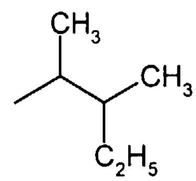 |
| Ia-23 | 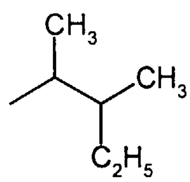 | $OCH_3$ |
| Ia-24 | $CH_3$ | 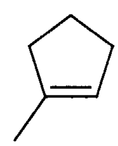 |
| Ia-25 | $CH_3$ | 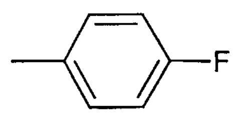 |
| Ia-26 | $CH_2CH_2OC_2H_5$ | $CH_2CH_2OC_2H_5$ |
| Ia-27 | 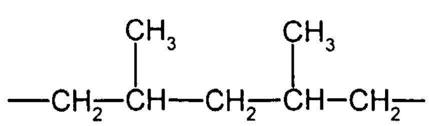 | |

**Tabelle** (Fortsetzung)

| Nr. | $R^1$ | $R^2$ |
|---|---|---|
| Ia-28 | $CH_3$ | $CH_2C_6H_5$ |
| Ia-29 | $CH_3$ | 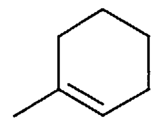 |
| Ia-30 | $CH_3$ | 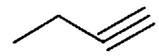 |
| Ia-31 | $CH(CH_3)_2$ | 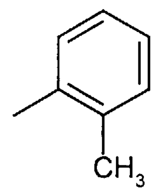 |
| Ia-32 | $CH_3$ | 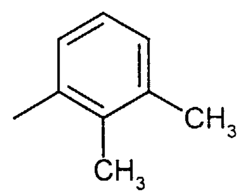 |
| Ia-33 | | 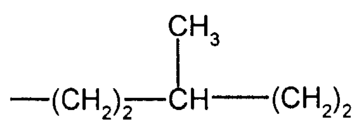 |
| Ia-34 | $C_2H_5$ | 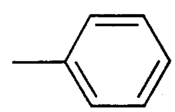 |
| Ia-35 | | 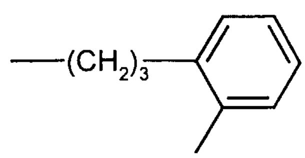 |
| Ia-36 | | 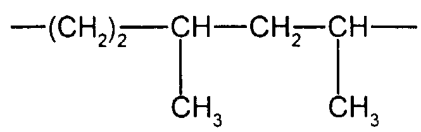 |
| Ia-37 | | 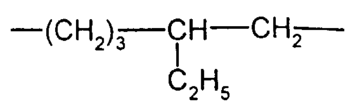 |

**Tabelle** (Fortsetzung)

| Nr. | $R^1$ | $R^2$ |
|---|---|---|
| Ia-38 | $CH(CH_3)_2$ | 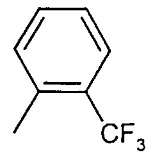 |
| Ia-39 | $CH(CH_3)_2$ | 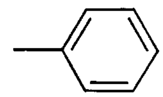 |
| Ia-40 | $CH(CH_3)_2$ | 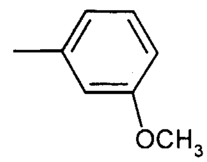 |

2. Verfahren zur Herstellung von Verbindungen der Formel (Ia) gemäß Anspruch 1, dadurch gekennzeichnet, daß man 3-Fluor-5-methylsulfonyl-1,2,4-thiadiazol der Formel (II-1)

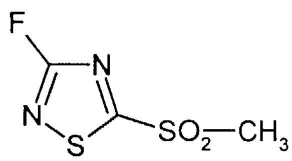

(II-1)

mit Hydroxyacetamiden der allgemeinen Formel (III)

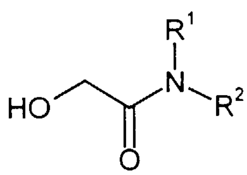

(III),

in welcher

$R^1$ und $R^2$ die oben angegebene Bedeutung haben,

gegebenenfalls in Gegenwart eines Verdünnungsmittels, gegebenenfalls in Gegenwart eines Säurebindemittels und gegebenenfalls in Gegenwart eines Katalysators umsetzt.

3. Herbizide Mittel, gekennzeichnet durch einen Gehalt an mindestens einer Verbindung gemäß Anspruch 1.

4. Verwendung von Verbindungen gemäß Anspruch 1 zur Bekämpfung von unerwünschtem Pflanzenwachstum.

5. Verfahren zur Bekämpfung von Unkräutern, dadurch gekennzeichnet, daß man Verbindungen gemäß Anspruch

1 auf die Unkräuter oder ihren Lebensraum einwirken läßt.

**6.** Verfahren zur Herstellung von herbiziden Mitteln, dadurch gekennzeichnet, daß man Verbindungen gemäß Anspruch 1 mit Streckmitteln und/oder oberflächenaktiven Mitteln vermischt.

**7.** 3-Fluor-5-methylthio-1,2,4-thiadiazol der Formel (IV-1)

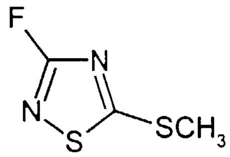

(IV-1).

**Claims**

**1.** N-methyl-N-phenyl-2-(3-fluoro-1,2,4-thiadiazol-5-yl-oxy)-acetamide of the formula (Ia-I)

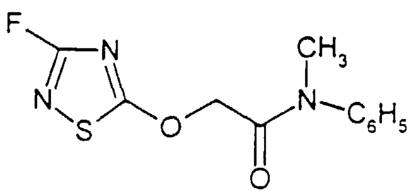

(Ia-I)

and the (3-fluoro-1,2,4-thiadiazol-5-yl-oxy)-acetamides of the formula (Ia)

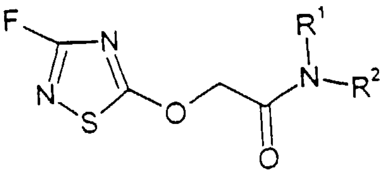

(Ia)

in which $R^1$ and $R^2$ in each case represent the radicals mentioned in the table below:

**Table**

| No. | $R^1$ | $R^2$ |
|---|---|---|
| Ia-2 | | $-(CH_2)_6-$ |
| Ia-3 | $CH_2-CH=CH_2$ | $CH_2-CH=CH_2$ |
| Ia-4 | $CH(CH_3)_2$ | $OCH_2CH_2OC_2H_5$ |
| Ia-5 | $CH(CH_3)_2$ | 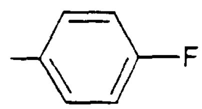 |
| Ia-6 | | 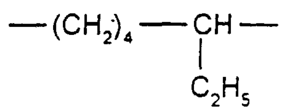 |
| Ia-7 | $CH_3$ | 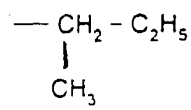 |
| Ia-8 | $n-C_3H_7$ | $n-C_3H_7$ |
| Ia-9 | | $-(CH_2)_5-$ |
| Ia-10 | $CH_2-CH=CH_2$ | $CH_2-CH=CH_2$ |
| Ia-11 | $CH(CH_3)_2$ | $OCH(CH_3)_2$ |
| II-12 | $CH_3$ | 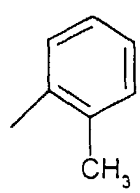 |
| Ia-13 | $C_2H_5$ | $C_2H_5$ |
| Ia-14 | $C_2H_5$ | 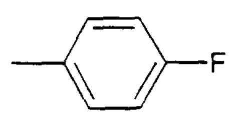 |
| Ia-15 | | 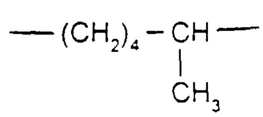 |
| Ia-16 | | 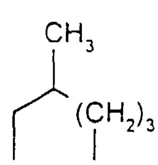 |

**Table** (continued)

| No. | $R^1$ | $R^2$ |
|---|---|---|
| Ia-17 | $CH_3$ | n-$C_4H_9$ |
| Ia-18 | $CH_3$ | 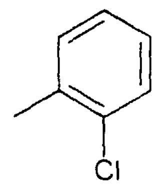 |
| Ia-19 | $C_2H_5$ | $C_6H_5$ |
| Ia-20 | n-$C_4H_9$ | n-$C_4H_9$ |
| Ia-21 | $CH_3$ | 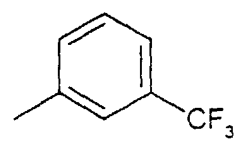 |
| Ia-22 | $CH_3$ | 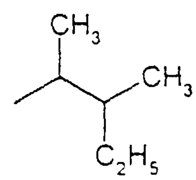 |
| Ia-23 | 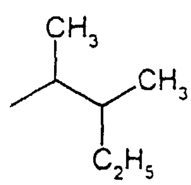 | $OCH_3$ |
| Ia-24 | $CH_3$ | 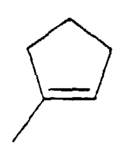 |
| Ia-25 | $CH_3$ | 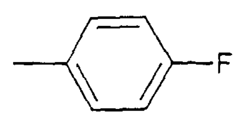 |
| Ia-26 | $CH_2CH_2OC_2H_5$ | $CH_2CH_2OC_2H_5$ |
| Ia-27 | 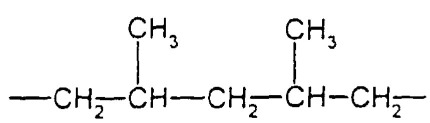 | |

**Table** (continued)

| No. | $R^1$ | $R^2$ |
|---|---|---|
| Ia-28 | $CH_3$ | $CH_2C_6H_5$ |
| Ia-29 | $CH_3$ | 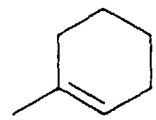 |
| Ia-30 | $CH_3$ | 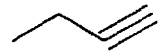 |
| Ia-31 | $CH(CH_3)_2$ | 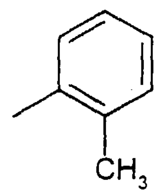 |
| Ia-32 | $CH_3$ | 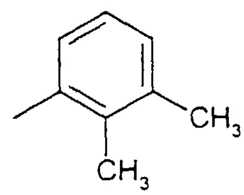 |
| Ia-33 | 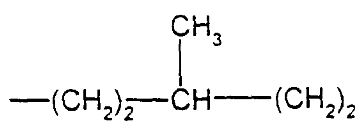 | |
| Ia-34 | $C_2H_5$ | 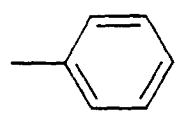 |
| Ia-35 | 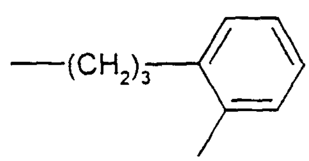 | |
| Ia-36 | 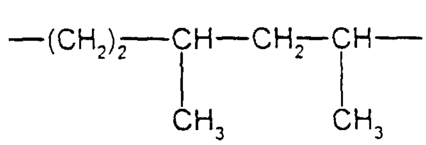 | |
| Ia-37 | 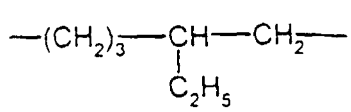 | |

**Table** (continued)

| No | $R^1$ | $R^2$ |
|---|---|---|
| Ia-38 | $CH(CH_3)_2$ | 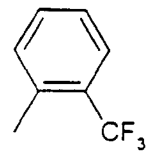 |
| Ia-39 | $CH(CH_3)_2$ | 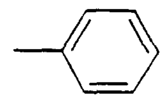 |
| Ia-40 | $CH(CH_3)_2$ | 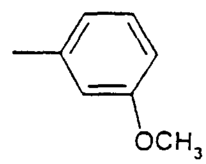 |

**2.** Process for preparing compounds of the formula (Ia) according to Claim 1, characterized in that 3-fluoro-5-methylsulphonyl-1,2,4-thiadiazol of the formula (II-1)

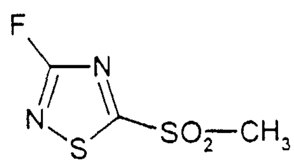

(II-1)

is reacted with hydroxyacetamides of the general formula (III)

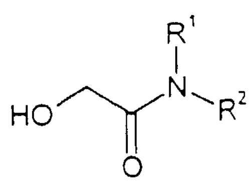

(III)

in which

$R^1$ and $R^2$ are as defined above,

if appropriate in the presence of a diluent, if appropriate in the presence of an acid binder and if appropriate in the presence of a catalyst.

**3.** Herbicidal compositions, characterized in that they comprise at least one compound according to Claim 1.

**4.** Use of compounds according to Claim 1 for controlling undesirable vegetation.

**5.** Method for controlling weeds, characterized in that compounds according to Claim 1 are allowed to act on the weeds or their habitat.

**6.** Process for preparing herbicidal compositions, characterized in that compounds according to Claim 1 are mixed with extenders and/or surfactants.

**7.** 3-Fluoro-5-methylthio-1,2,4-thiadiazole of the formula (IV-1)

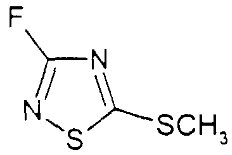

(IV-1).

**Revendications**

**1.** N-méthyl-N-phényl-2-(3-fluoro-1,2,4-thiadiazole-5-yloxy)-acétamide de formule (Ia-1)

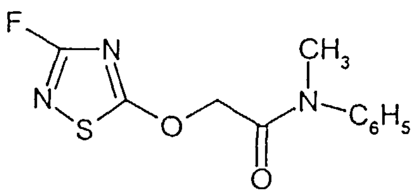

(Ia-1)

ainsi que les (3-fluoro-1,2,4-thiadiazole-5-yloxy)-acétamides de formule (Ia)

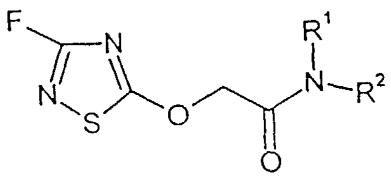

(Ia)

dans laquelle $R^1$ et $R^2$ représentent chacun les restes mentionnés dans le tableau suivant :

## TABLEAU

| Nr. | $R^1$ | $R^2$ |
|---|---|---|
| Ia-2 | | $-(CH_2)_6-$ |
| Ia-3 | $CH_2\text{-}CH{=}CH_2$ | $CH_2\text{-}CH{=}CH_2$ |
| Ia-4 | $CH(CH_3)_2$ | $OCH_2CH_2OC_2H_5$ |
| Ia-5 | $CH(CH_3)_2$ | 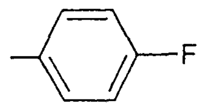 |
| Ia-6 | | 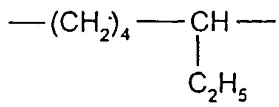 |
| Ia-7 | $CH_3$ | 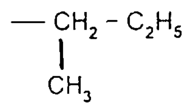 |
| Ia-8 | $n\text{-}C_3H_7$ | $n\text{-}C_3H_7$ |
| Ia-9 | | $-(CH_2)_5-$ |
| Ia-10 | $CH_2\text{-}CH{=}CH_2$ | $CH_2\text{-}CH{=}CH_2$ |
| Ia-11 | $CH(CH_3)_2$ | $OCH(CH_3)_2$ |
| II-12 | $CH_3$ | 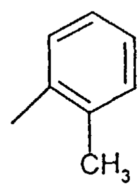 |
| Ia-13 | $C_2H_5$ | $C_2H_5$ |
| Ia-14 | $C_2H_5$ | 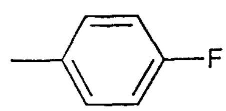 |
| Ia-15 | | 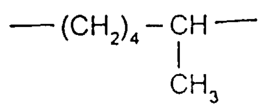 |
| Ia-16 | | 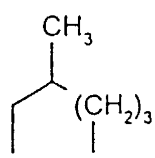 |

**TABLEAU (Suite)**

| Nr. | $R^1$ | $R^2$ |
|---|---|---|
| Ia-17 | $CH_3$ | n-$C_4H_9$ |
| Ia-18 | $CH_3$ | 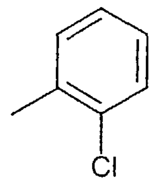 |
| Ia-19 | $C_2H_5$ | $C_6H_5$ |
| Ia-20 | n-$C_4H_9$ | n-$C_4H_9$ |
| Ia-21 | $CH_3$ | 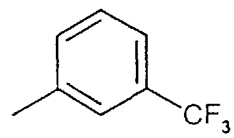 |
| Ia-22 | $CH_3$ | 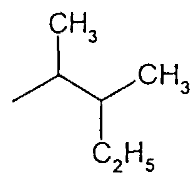 |
| Ia-23 | 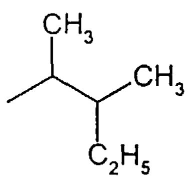 | $OCH_3$ |
| Ia-24 | $CH_3$ | 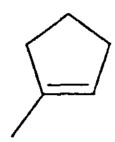 |
| Ia-25 | $CH_3$ | 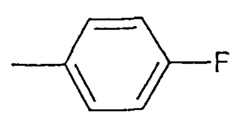 |
| Ia-26 | $CH_2CH_2OC_2H_5$ | $CH_2CH_2OC_2H_5$ |
| Ia-27 | 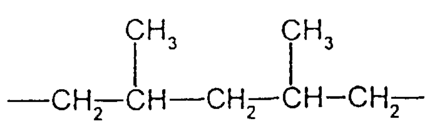 | |

**TABLEAU (Suite)**

| Nr. | $R^1$ | $R^2$ |
|---|---|---|
| Ia-28 | $CH_3$ | $CH_2C_6H_5$ |
| Ia-29 | $CH_3$ | 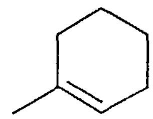 |
| Ia-30 | $CH_3$ | 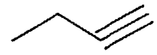 |
| Ia-31 | $CH(CH_3)_2$ | 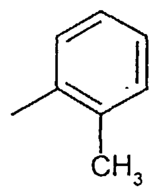 |
| Ia-32 | $CH_3$ | 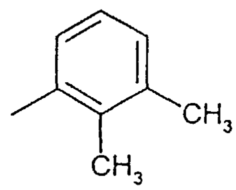 |
| Ia-33 | 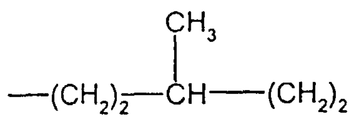 | |
| Ia-34 | $C_2H_5$ | 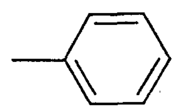 |
| Ia-35 | 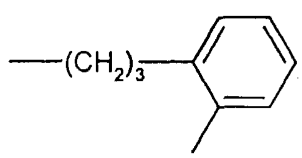 | |
| Ia-36 | 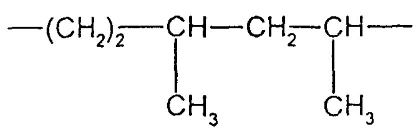 | |
| Ia-37 | 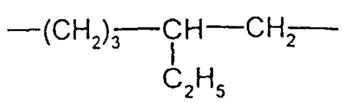 | |

**TABLEAU (Suite)**

| Nr. | $R^1$ | $R^2$ |
|---|---|---|
| Ia-38 | $CH(CH_3)_2$ | 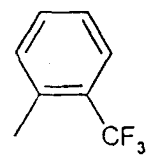 |
| Ia-39 | $CH(CH_3)_2$ | 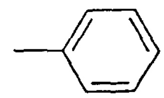 |
| Ia-40 | $CH(CH_3)_2$ | 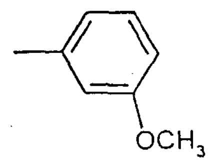 |

**2.** Procédé de production de composés de formule (Ia) suivant la revendication 1, caractérisé en ce qu'on fait réagir le 3-fluoro-5-méthylsulfonyl-1,2,4-thiadiazole de formule (II-1)

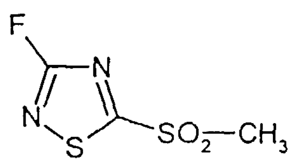

(II-1)

avec des hydroxyacétamides de formule générale (III)

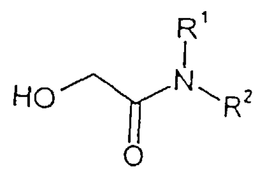

(III),

dans laquelle

$R^1$ et $R^2$ ont la définition indiquée ci-dessus,

le cas échéant en présence d'un diluant, éventuellement en présence d'un accepteur d'acide et en la présence éventuelle d'un catalyseur.

**3.** Compositions herbicides, caractérisées par une teneur en au moins un composé suivant la revendication 1.

**4.** Utilisation de composés suivant la revendication 1 pour combattre une croissance végétale indésirable.

**5.** Procédé pour combattre des mauvaises herbes, caractérisé en ce qu'on fait agir des composés suivant la revendication 1 sur les mauvaises herbes ou sur leur milieu.

**6.** Procédé de préparation de compositions herbicides, caractérisé en ce qu'on mélange des composés suivant la revendication 1 avec des diluants et/ou des agents tensioactifs.

**7.** Le 3-fluoro-5-méthylthio-1,2,4-thiadiazole de formule (IV-1)

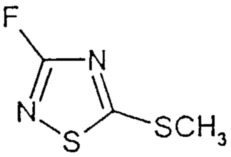

(IV-1).